# EUROPÄISCHE PATENTANMELDUNG

(11) **EP 2 545 888 A1**
(43) Veröffentlichungstag der Anmeldung: **16.01.2013**
(21) Anmeldenummer: 12005059.6
(22) Anmeldetag: 09.07.2012
(51) Int. Cl.: A61F 5/37

(54) **Fixierbandage mit zwei Hauptgurten**

(30) Priorität: 12.07.2011 DE 202011103315 U
(71) Anmelder: Wysozki, Roman, 22763 Hamburg (DE)
(72) Erfinder: Wysozki, Roman, 22763 Hamburg (DE)
(74) Vertreter: Hauck Patent- und Rechtsanwälte

(57) **Zusammenfassung**

Fixierbandage zur Fixierung eines Patienten auf einem Bett oder einem sonstigen Lager mit zwei Betthalterungen zur Befestigung an einem Bettgestell, zwei Hauptgurten, die jeweils ein erstes Ende, das mit einer der Betthalterungen verbunden ist, und ein freies Ende aufweisen, wobei die freien Enden Befestigungseinrichtungen zur lösbaren Verbindung der freien Enden mitelnander aufweisen und die Hauptgurte jeweils mindestens einen ersten Materialstreifen aufweisen, der durchgängig vom ersten Ende bis zum freien Ende des Hauptgurts verläuft, und einem ersten Verbindungsgurt, der die beiden Hauptgurte miteinander verbindet, so dass erste Gurtabschnitte der Hauptgurte, die sich jeweils von einem der ersten Enden des jeweiligen Hauptgurts bis zu dem ersten Verbindungsgurt erstrecken, und der erste Verbindungsgurt mit Hilfe der Betthalterungen über eine Liegefläche des Betts gespannt und die freien Enden der beiden Hauptgurte um den Leib des Patienten herumgeführt und miteinander verbunden werden können.

## Beschreibung

Die Erfindung betrifft eine Fixierbandage zur Fixierung eines Patienten auf einem Bett oder einem sonstigen Lager.

Aus dem Gebrauchsmuster DE 203 17 701 U1 ist eine Fixierbandage bekannt geworden, die einen flach auf das Bett aufzulegenden Bettgurt aufweist, der zur Befestigung an einem Bettgestell des Betts an jedem seiner beiden Enden eine Betthalterung aufweist. Die Betthalterungen werden jeweils schlaufenförmig um einen Holm des Bettgestells herumgeschlungen und mit einer geeigneten Verriegelung, beispielsweise einem Magnetschloss, geschlossen. Dadurch ist der Bettgurt an dem Bett befestigt. An dem Bettgurt ist ein Leibgurt befestigt, der ringförmig um die Taille des Patienten herumgelegt und mit einer weiteren Verriegelung geschlossen werden kann. Dadurch ist der Patient an der Taille auf dem Bett fixiert. Dies schützt ihn vor einem Herausfallen aus dem Bett, ohne seine Bewegungsfreiheit insbesondere an den Händen und Füßen unnötig einzuschränken. Bei derartigen Fixierbandagen erhält der Patient zusätzliche Bewegungsfreiheit dadurch, dass der Leibgurt nicht unmittelbar mit dem Bettgurt vernäht ist, sondern mit Hilfe sich überkreuzender Bänder. Dies ist in Grundzügen bereits in der Offenlegungsschrift DE 1 953 842 beschrieben. Durch die sich überkreuzenden Bänder kann der ringförmig geschlossene Leibgurt in einem gewissen Bereich auf dem Bettgurt abrollen und somit dem Patienten ermöglichen, sich auf die Seite zu drehen.

Dieser zusätzliche Bewegungsspielraum kann sich unter Umständen jedoch auch als nachteilig erweisen, insbesondere wenn der Patient infolge einer seitlichen Drehung seine Beine seitlich über eine Bettkante hinauslehnt, zu tief in den Leibgurt hineinrutscht und dann von dem Leibgurt in seiner Atmung beeinträchtigt wird.

Es ist daher bekannt geworden, die seitliche Bewegungsfreiheit durch einen zusätzlichen Quergurt, der sich von der Oberseite des Leibgurts seitlich zu den Betthalterungen erstreckt, gezielt einzuschränken, wie im Einzelnen in der Druckschrift DE 20 2006 009 390 U1 beschrieben.

Eine andere Form einer zusätzlichen seitlichen Fixierung ist in der bereits genannten Druckschrift DE 20 317 701 U1 beschrieben. Hier werden an beiden Seiten des Leibgurts zusätzliche Seitenbefestigungen angebracht, die mit den Betthalterungen verbunden werden. Für eine sichere Verwendung von Fixierbandagen mit derartigen zusätzlichen Seitenbefestigungen müssen diese immer gewissenhaft und korrekt angelegt werden. Außerdem besteht ein Risiko, dass der Patient mit einem Körperteil, insbesondere mit dem Kopf, unter eine der Seitenbefestigungen gerät.

Um die zusätzlichen Seitenbefestigungen einzusparen und die Handhabung der Fixierbandage zu vereinfachen, schlägt die Druckschrift DE 20 2006 009 392 U1 vor, den Leibgurt und den Bettgurt unmittelbar miteinander zu vernähen. Hierdurch kann zwar auf die Seitenbefestigungen verzichtet werden, der Patient wird jedoch in seiner Bewegungsfreiheit stärker eingeschränkt und es ist nicht länger möglich, ihm durch gezieltes Lockern der Seitenbefestigungen ein gewünschtes Maß an Bewegungsfreiheit einzuräumen.

Außerdem schlägt die letztgenannte Druckschrift zwecks Materialeinsparung vor, im Verbindungsbereich von Bettgurt und Leibgurt einen Längsabschnitt des Leibgurts oder des Bettgurts auszusparen. Entweder kann der Leibgurt zweigeteilt werden und die beiden Teile des Leibgurts können in einem Abstand voneinander auf einem durchgängigen Bettgurt befestigt werden. Alternativ kann der Bettgurt geteilt werden und zwei Enden der beiden Bettgurtteile können in einem Abstand voneinander an einem durchgängigen Leibgurt befestigt werden.

Davon ausgehend ist es die Aufgabe der Erfindung, eine Fixierbandage zur Fixierung eines Patienten auf einem Bett oder einem sonstigen Lager zur Verfügung zu stellen, die besonders einfach und robust aufgebaut ist.

Diese Aufgabe wird gelöst durch die Fixierbandage mit den Merkmalen des Anspruchs 1. Vorteilhafte Ausgestaltungen sind in den sich anschließenden Unteransprüchen angegeben.

Die Fixierbandage dient zur Fixierung eines Patienten auf einem Bett oder einem sonstigen Lager und hat
● zwei Betthalterungen zur Befestigung an einem Bettgestell,
● zwei Hauptgurte, die jeweils ein erstes Ende, das mit einer der Betthalterungen verbunden ist, und ein freies Ende aufweisen, wobei die freien Enden Befestigungseinrichtungen zur lösbaren Verbindung der freien Enden miteinander aufweisen und die Hauptgurte jeweils mindestens einen ersten Materialstreifen aufweisen, der durchgängig vom ersten Ende bis zum freien Ende des Hauptgurts verläuft, und
● einen ersten Verbindungsgurt, der die beiden Hauptgurte miteinander verbindet, so dass erste Gurtabschnitte der Hauptgurte, die sich jeweils von einem der ersten Enden des jeweiligen Hauptgurts bis zu dem ersten Verbindungsgurt erstrecken, und der erste Verbindungsgurt mit Hilfe der Betthalterungen über eine Liegefläche des Betts gespannt und die freien Enden der beiden Hauptgurte um den Leib des Patienten herumgeführt und miteinander verbunden werden können.

Die Fixierbandage dient zur Fixierung eines Patienten auf einem Bett oder einem sonstigen Lager. Mit Fixierung ist einerseits eine Ruhigstellung verwirrter, unkooperativer oder nicht zurechnungsfähiger Patienten gemeint. Eingezogen ist darüber hinaus eine Lagerung von Patienten in einer bevorzugten Haltung aus anderen Gründen, beispielsweise zur Dekubitus-Prophylaxe. Das sonstige Lager kann beispielsweise eine Trage oder eine Liege sein, etwa für einen Krankentransport. Soweit in den Anspruchsmerkmalen auf Elemente des Betts wie das Bettgestell oder eine Liegefläche Bezug genommen wird, geschieht dies der Einfachheit halber stellvertretend für entsprechende Komponenten sonstiger Lager. Beispielsweise kann das Bettgestell im Falle einer Trage ein Rahmen der Trage sein und die Liegefläche des Betts kann eine Liegefläche der Trage sein, usw.

Die Betthalterungen können Gurte sein, die zur Befestigung an einem Bettgestell um einen Bettholm des Bettgestells (oder, beispielsweise, zur Befestigung an einer Trage um einen Träger eines Rahmens der Trage) herumgeführt werden. Die von jeder Betthalterung auf diese Weise gebildete Schlaufe wird mit einer geeigneten Verriegelung geschlossen. Diese ist beispielsweise ein Magnetschloss, das einen Sockel mit einem Teller und einem Schaft und einen Schließknopf aufweist. Der Schaft wird durch zwei Ösen in der Betthalterung hindurch gesteckt und mit dem Schließknopf versehen. Der Teller des Sockels kann sich unterhalb der Ösen in einer Tasche der Betthalterung befinden.

Die beiden freien Enden der Hauptgurte weisen Befestigungseinrichtungen auf, so dass sie lösbar miteinander verbunden werden können. Die Befestigungseinrichtungen können beispielsweise Ösen sein, so dass die beiden Hauptgurte beispielsweise mit einem Magnetschloss, das durch die Ösen hindurchgeführt wird, verbindbar sind. Die freien Enden der beiden Hauptgurte können um den Leib des Patienten herumgeführt und auf zum Beispiel auf diese Weise miteinander verbunden werden. Abschnitte der Hauptgurte und der erste Verbindungsgurt umschließen dann den Patienten ringförmig und entsprechen ihrer Funktion nach einem Leibgurt.

Dass einzelne Elemente der Fixierbandage, insbesondere die Betthalterungen, Gurte oder Enden einzelner Gurte verbunden sind, wie vorstehend im einzelnen angegeben, bedeutet, dass Zugkräfte von dem einen Element auf das damit verbundene Element übertragen werden können. Die Verbindung kann fest ausgeführt sein, beispielsweise durch Vernähen der beiden Elemente, insbesondere in einem Längsabschnitt, indem sich die jeweiligen Elemente überlappen. Hier können die Abschnitte insbesondere großflächig, das heißt durch mehrere voneinander beabstandete Nähte, vernäht sein. Die Verbindung kann jedoch auf lösbar ausgeführt sein, etwa durch Schlaufen oder Ösen, durch die Enden der Elemente hindurchgeführt oder an denen sie in sonstiger Weise befestigt sind. Darüber hinaus können miteinander in der beanspruchten Weise verbundene Elemente auch von einem einteilig ausgebildeten Gurt gebildet sein.

Bei der Erfindung wird die für den Stand der Technik prägende, grundsätzliche konstruktive Trennung zwischen einem Bettgurt und einem Leibgurt aufgegeben. Stattdessen wird zu beiden Seiten des Patienten jeweils ein Hauptgurt angeordnet, der gleichzeitig Teile der Funktion eines herkömmlichen Leibgurts und Teile der Funktion eines herkömmlichen Bettgurts übernimmt. Die beiden Hauptgurte weisen jeweils mindestens einen ersten Materialstreifen auf, der durchgängig vom ersten Ende bis zum freien Ende des betreffenden Hauptgurts verläuft. Insbesondere können die Hauptgurte insgesamt durchgängig zwischen den genannten Enden ausgeführt sein, gegebenenfalls bestehend aus mehreren unterschiedlichen Lagen und/oder Materialien. Die durchgängigen Materialstreifen erlauben eine dauerhafte Einleitung von Zugkräften vom Körper des Patienten bis in die Betthalterungen. Wegen des durchgängigen ersten Materialstreifens gibt es dabei entlang der Länge jedes Hauptgurts keine empfindlichen Verbindungsstellen zwischen einem Leibgurt und einem Bettgurt, wie bei den eingangs erläuterten, aus dem Stand der Technik bekannten Konstruktion. Die Fixierbandage ist daher gleichzeitig besonders einfach aufgebaut und besonders robust und langlebig. Die Verwendung der Hauptgurte ermöglicht auch eine besonders einfache und materialsparende Herstellung, denn die Anzahl der Verbindungen zwischen unterschiedlichen Gurten reduziert sich und es gibt entsprechend weniger sich überlappende Bereiche.

In einer Ausgestaltung weisen die Hauptgurte eine erste Breite auf und der mindestens eine erste Materialstreifen ist ein erstes Gurtmaterial mit der ersten Breite. Sofern jetzt und im Folgenden von unterschiedlichen Gurtmaterialien die Rede ist, etwa von einem ersten und einem zweiten Gurtmaterial, bezeichnet der Begriff Gurtmaterial jeweils einen bestimmten Gurttyp, der aus einem bestimmten Werkstoff, insbesondere Fasern oder Fäden, etwa Nylon- oder sonstigen Kunstfasern oder Baumwoll- oder Leinenfäden, besteht, bestimmte Abmessungen, insbesondere eine bestimmte Breite und Stärke, und einen bestimmten Gewebetyp (oder ein Gewirk oder Geflecht) aufweist. Derartige Gurtmaterialien sind in den unterschiedlichsten Varianten insbesondere von der Rolle erhältlich. Demnach können sich im Sinne der hier verwendeten Begriffe unterschiedliche Gurtmaterialien lediglich in einer der genannten Eigenschaften, etwa in der Breite, unterscheiden. Grundsätzlich kann der erste Materialstreifen auch schmaler ausgeführt sein als der zugehörige Hauptgurt. Eine besonders robuste Konstruktion ergibt sich, wenn der erste Materialstreifen aus einem sich über die gesamte Breite der Hauptgurte erstreckenden, relativ breiten Gurtmaterial besteht und/oder der Hauptgurt im Wesentlichen aus dem ersten Materialstreifen gefertigt ist.

In einer Ausgestaltung weist der erste Verbindungsgurt die gleiche Breite auf wie die Hauptgurte. Dies trägt insbesondere dann zu einem hohen Tragekomfort bei, wenn der erste Verbindungsgurt am Rücken des Patienten anliegt. Grundsätzlich umschließt der erste Verbindungsgurt gemeinsam mit Teilen der Hauptgurte den Leib des Patienten ringförmig. Eine gleichmäßige Breite kann daher Beeinträchtigungen insbesondere im Bereich der Verbindungen des ersten Verbindungsgurts zu den Hauptgurten vermeiden.

In einer Ausgestaltung weist der erste Verbindungsgurt einen Streifen des ersten Gurtmaterials auf. Insbesondere kann der erste Verbindungsgurt aus einem solchen Streifen bestehen. Die Verbindung gleichartiger Gurtmaterialien ist hinsichtlich der Verarbeitung und Festigkeit vorteilhaft.

In einer Ausgestaltung verläuft der mindestens eine erste Materialstreifen durchgängig von einem freien Ende eines der Hauptgurte bis zu einem freien Ende einer der Betthalterungen. Es werden also auch die Betthalterungen zumindest teilweise von dem durchgängigen, ersten Materialstreifen gebildet, so dass auch der Übergang vom ersten Ende des jeweiligen Hauptgurts zur sich daran anschließenden Betthalterung eine erhöhte Festigkeit aufweist.

In einer Ausgestaltung weist der erste Verbindungsgurt mindestens einen zweiten Materialstreifen auf, der durchgängig bis zu einer der beiden Betthalterungen verläuft. Bevorzugt kann der mindestens eine zweite Materialstreifen durchgängig von der auf einer Seite des Patienten angeordneten Betthalterung zu der auf der gegenüberliegenden Seite des Patienten angeordneten Betthalterung verlaufen. Grundsätzlich ist bei der Erfindung vorgesehen, dass die auftretenden Zugkräfte im Wesentlichen über die beiden Hauptgurte in die Betthalterungen eingeleitet werden. Ein durchgängiger zweiter Materialstreifen als Teil oder Hauptbestandteil des ersten Verbindungsgurts ermöglicht eine zusätzliche Krafteinleitung in die Betthalterungen ausgehend vom ersten Verbindungsgurt. Dies entlastet die Verbindungen des ersten Verbindungsgurts zu den Hauptgurten.

In einer Ausgestaltung verläuft der zweite Materialstreifen von einem Befestigungspunkt des ersten Verbindungsgurts an einem Hauptgurt entlang dieses Hauptgurts und ist mit diesem vernäht. Insbesondere kann der zweite Materialstreifen an einer auf der Liegefläche aufliegenden Unterseite des betreffenden Hauptgurts angeordnet sein. Er kann über die gesamte Länge des auf der Liegefläche aufliegenden Abschnitts des Hauptgurts mit diesem vernäht sein. Es ergibt sich eine besonders feste Verbindung über einen relativ großen Längsabschnitt. Außerdem wird die Anordnung des zweiten Materialstreifens gegenüber dem Hauptgurt fixiert, insbesondere wenn sich der zweite Materialstreifen bis zu einer Betthalterung erstreckt. Dadurch wird vermieden, dass der Patient beispielsweise mit einer Hand zwischen den zweiten Materialstreifen und den Hauptgurt gelangt.

In einer Ausgestaltung ist ein zweiter Verbindungsgurt vorhanden, der die beiden Hauptgurte im Rücken des Patienten miteinander verbindet. Wie in einer weiteren Ausgestaltung vorgesehen, sind dabei die Enden des zweiten Verbindungsgurts jeweils in einem entlang der Hauptgurte betrachtet größeren Abstand von den ersten Enden des jeweiligen Hauptgurts entfernt an dem Hauptgurt befestigt als die Enden des ersten Verbindungsgurts. In diesem Fall wird der Leib des Patienten von dem im Rücken des Patienten befindlichen zweiten Verbindungsgurt und den sich daran bis zu den freien Enden der Hauptgurte erstreckenden Hauptgurtabschnitten ringförmig umschlossen, während der erste Verbindungsgurt und die sich daran in Richtung zu den Betthalterungen hin anschließenden Abschnitte der Hauptgurte auf der Liegefläche des Betts aufliegen. Die zwischen den Befestigungsbereichen des ersten Verbindungsgurts mit den Hauptgurten und des zweiten Verbindungsgurts mit den Hauptgurten befindlichen Längsabschnitte der beiden Hauptgurte stellen eine Verbindung zwischen den beiden funktional an dem Bettgurt und an dem Leibgurt entsprechenden Elementen der Fixierbandage her, die dem Patienten eine gewisse Bewegungsfreiheit einschließlich der Möglichkeit, sich ein Stück weit auf die Seite zu drehen, einräumt. Gleichzeitig bleibt die optimale Krafteinleitung über die Hauptgurte vom Körper des Patienten bis zu den Betthalterungen hin erhalten. Die die Verbindung herstellenden Längsabschnitte der Hauptgurte können eine Länge beispielsweise im Bereich von 10 cm bis 25 cm aufweisen, insbesondere im Bereich von 15 cm bis 20 cm.

In einer Ausgestaltung weist der zweite Verbindungsgurt die gleiche Breite auf wie die beiden Hauptgurte. Es ergibt sich wegen der dann gleichmäßigen Breite der den Leib des Patienten umschließenden Gurtabschnitte ein optimaler Tragekomfort.

In einer Ausgestaltung weist der zweite Verbindungsgurt mindestens ein Stück des ersten Gurtmaterials auf. Dies ist hinsichtlich der Festigkeit und Verarbeitung der Verbindung zwischen dem zweiten Verbindungsgurt und den Hauptgurten vorteilhaft.

In einer Ausgestaltung weist der erste Verbindungsgurt eine zweite Breite auf, die geringer ist als die erste Breite. Eine geringere Breite des ersten Verbindungsgurts beeinträchtigt den Tragekomfort nicht, wenn am Körper des Patienten im Wesentlichen der zweite Verbindungsgurt anliegt. Gleichzeitig ist ein schmalerer erster Verbindungsgurt zur Aufnahme der in diesem Bereich auftretenden Belastungen ausreichen.

In einer Ausgestaltung ist der zweite Materialstreifen ein zweites Gurtmaterial mit der zweiten Breite. Der zweite Verbindungsgurt kann teilweise oder vollständig von diesem zweiten Gurtmaterial gebildet sein.

In einer Ausgestaltung weisen die beiden Betthalterungen die zweite Breite auf und mindestens ein Stück des zweiten Gurtmaterials. Die Verwendung desselben Gurtmaterials für die Betthalterungen und den ersten Verbindungsgurt ist besonders wirtschaftlich.

In einer Ausgestaltung verläuft der zweite Materialstreifen durchgängig bis zu einem freien Ende einer der beiden Betthalterungen. Er kann auch durchgängig von einem freien Ende einer links des Patienten angeordneten Betthalterung bis zu einem freien Ende einer rechts des Patienten angeordneten Betthalterung verlaufen. Durch einen durchgängigen zweiten Materialstreifen wird die Krafteinleitung vom ersten Verbindungsgurt in die Betthalterungen verbessert. Außerdem vereinfacht sich die Fertigung der Fixierbandage.

In einer Ausgestaltung ist bzw. sind an dem ersten Verbindungsgurt oder an dem zweiten Verbindungsgurt ein Schrittgurt oder Oberschenkelmanschetten befestigt. Hierzu kann der Schrittgurt insbesondere unmittelbar mit dem ersten oder zweiten Gurtabschnitt vernäht sein, beispielsweise großflächig und/oder anhand mehrerer benachbarter Nähte. Der Schrittgurt kann auch mittelbar, d.h. beispielsweise über ein Verstärkungs- oder Zwischenstück, an dem ersten oder zweiten Verbindungsgurt angebracht sein. Es kann auch eine lösbare Befestigung, beispielsweise mit Hilfe einer Schlaufe oder Öse, vorgesehen sein. Der Schrittgurt kann ein freies Ende mit einer Befestigungseinrichtung aufweisen, beispielsweise mit mehreren voneinander beabstandeten Ösen, die eine Befestigung des freien Endes des Schrittgurts an den Hauptgurten, insbesondere im Bereich der beiden lösbar miteinander verbundenen freien Enden der Hauptgurte, ermöglicht. Der Schrittgurt kann ein Polster aufweisen, das zwischen den Beinen des Patienten angeordnet ist und den Tragekomfort verbessert und vor Verletzungen schützt. Der Schrittgurt hat die Wirkung, dass ein zu tiefes Hineinrutschen des Patienten in die Fixierbandage zuverlässig verhindert wird. Anstelle eines Schrittgurts können auch Oberschenkelgurte oder -manschetten verwendet werden, die ringförmig um jeweils einen Oberschenkel des Patienten herum angelegt werden und ebenfalls ein zu tiefes Hineinrutschen des Patienten in die Fixierbandage verhindern. Die Oberschenkelgurte oder -manschetten können auf die gleiche Art und Weise an dem ersten und/oder zweiten Verbindungsgurt befestigt werden wie für den Schrittgurt beschrieben.

In einer Ausgestaltung sind an den Hauptgurten Befestigungseinrichtungen zur Anbringung eines Brust- oder Schultergurts ausgebildet. Die Befestigungseinrichtungen können beispielsweise zwei in einem Abstand voneinander angeordnete Schlaufen oder Ösen sein, durch die weitere Gurte hindurchgeführt werden können. Ein auf diese Weise angebrachter Brust- oder Schultergurt kann ein Herausflüchten des Patienten aus der Fixierbandage nach oben verhindern. Alternativ kann der Brust- oder Schultergurt auch unlösbar an den Hauptgurten befestigt sein, insbesondere durch Vernähen. Hierzu kann der Brust- oder Schultergurt unmittelbar an den Hauptgurten oder gegebenenfalls über ein zusätzliches Verstärkungs- oder Zwischenstück daran angebracht sein.

In einer Ausgestaltung ist ein Verbindungsbereich zwischen einem der Hauptgurte und einem der Verbindungsgurte mit einem Materialstück verstärkt, das mit dem jeweiligen Hauptgurt und dem jeweiligen Verbindungsgurt vernäht ist. Das Materialstück kann beispielsweise rechteckig oder oval sein und beispielsweise aus einem Gurtmaterial bestehen. Eine derartige Verstärkung eines Verbindungsbereichs erhöht die Belastbarkeit und Lebensdauer der Fixierbandage.

Die Erfindung wird nachfolgend anhand von zwei in fünf Figuren dargestellten Ausführungsbeispielen näher erläutert. Es zeigen:
- Fig. 1: ein erstes Ausführungsbeispiel einer erfindungsgemäßen Fixierbandage in an einen Patienten angelegtem Zustand in einer vereinfachten Schnittdarstellung,
- Fig. 2: die Fixierbandage aus Fig. 1 in einer perspektivischen Ansicht ohne Patienten,
- Fig. 3: ein zweites Ausführungsbeispiel einer erfindungsgemäßen Fixierbandage in an einen Patienten angelegtem Zustand in einer Schnittdarstellung,
- Fig. 4: die Fixierbandage aus Fig. 3 in einer perspektivischen Ansicht,
- Fig. 5: einen Ausschnitt der Fixierbandage der Figuren 3 und 4 in einer anderen perspektivischen Ansicht.

Für sich entsprechende Elemente werden in allen Figuren dieselben Bezugszeichen verwendet.

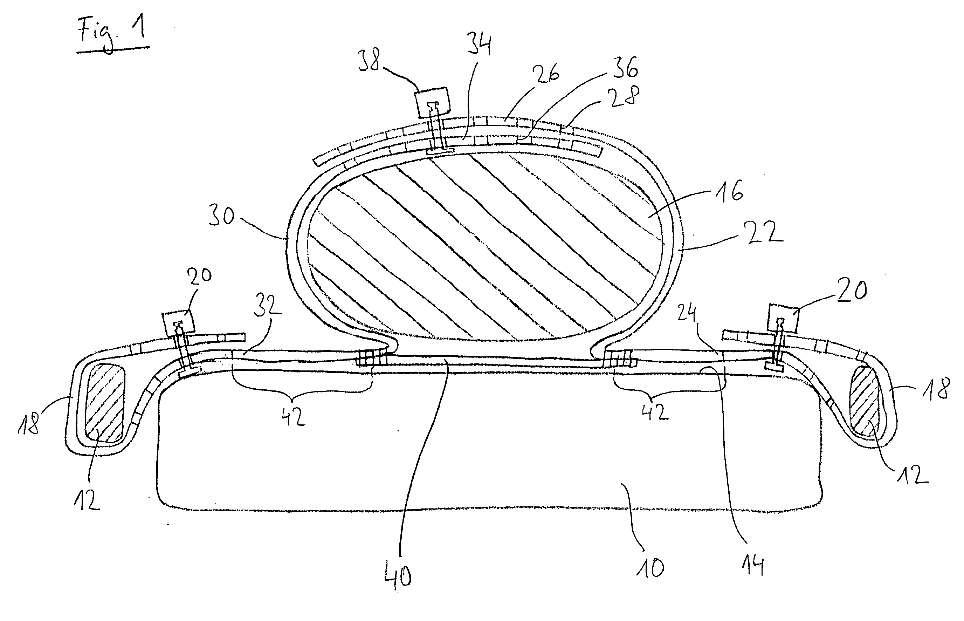

Fig. 1 zeigt im Querschnitt ein Bett mit Matratze 10 und zwei Holmen eines Bettgestells 12. Die Oberseite der Matratze 10 bildet eine Liegefläche 14, auf der ein auf dem Rücken liegender Patient 16 ruht. Der Abstand zwischen Patient 16 und Liegefläche 14 ist vergrößert dargestellt, um die dazwischen angeordneten Elemente der Fixierbandage besser erkennbar darstellen zu können. Der dargestellte Schnitt verläuft durch den Leib des Patienten im Bauchbereich und der Blick ist von unten, das heißt von den Füßen des Patienten aus, auf diese Schnittfläche gerichtet. Deshalb sind die rechts des Patienten befindlichen Elemente in der Figur links dargestellt und umgekehrt.

Die Fixierbandage weist zwei Betthalterungen 18 auf, die jeweils um einen Holm des Bettgestells 12 herumgeschlungen und mit einem Magnetschloss 20, das durch zwei Ösen der Betthalterungen 18 hindurchgeführt ist, befestigt sind.

Die Fixierbandage weist weiterhin einen linken Hauptgurt 22 auf. Der linke Hauptgurt 22 hat ein erstes Ende 24, das mit der linken Betthalterung 18 verbunden ist, und eine freies Ende 26 mit einer Befestigungseinrichtung in Form von mehreren Ösen 28. Entsprechend weist die Fixierbandage einen rechten Hauptgurt 30 auf mit einem ersten Ende 32, das mit der rechten Betthalterung 18 verbunden ist, und einem freien Ende 34 mit Ösen 36. Die Verbindungen zwischen den Betthalterungen und den Hauptgurten 22, 30 sind in der Figur nicht im Einzelnen dargestellt. Sie können auf unterschiedliche Weise hergestellt werden, etwa durch Vernähen überlappender Gurtabschnitte oder durch Verwendung durchgängiger Materialstreifen. Die freien Enden 26, 34 sind mittels eines Magnetschlosses 38, dass durch jeweils eine der Ösen 28, 36 hindurchgeführt ist, lösbar miteinander verbunden.

Ein erster Verbindungsgurt 40 verbindet die beiden Hauptgurte 22, 30 miteinander. Seine beiden Enden sind, wie durch jeweils fünf kleine parallele Linien angedeutet, mit jeweils einem der beiden Hauptgurte 22, 30 mit mehreren benachbarten Nähten vernäht.

Die Hauptgurte 22, 30 umfassen jeweils einen ersten Gurtabschnitt 42, der sich von einem der ersten Enden 24, 32 bis zu dem Verbindungsgurt 40 erstreckt und auf der Liegefläche 14 aufliegt. Der erste Verbindungsgurt 40 liegt ebenfalls auf der Liegefläche 14 auf. Der erste Verbindungsgurt 40 und die ersten Gurtabschnitte 42 sind mit Hilfe der Betthalterungen 18 über die Liegefläche 14 gespannt.

Die freien Enden 26, 34 der Hauptgurte 22, 30 sind um den Leib des Patienten 16 herumgeführt. Gemeinsam mit den seitlich des Patienten 16 angeordneten Abschnitten der Hauptgurte 22, 30 und dem ersten Verbindungsgurt 40 umschließen sie den Patienten 16 ringförmig.

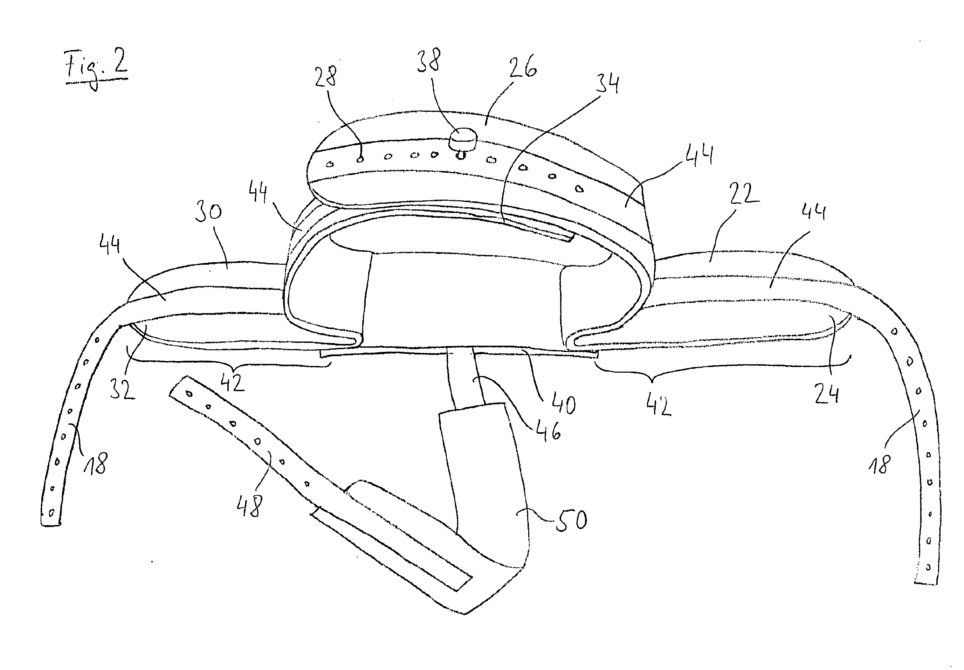

In der Figur 2 sind die bereits erläuterten Elemente der Fixierbandage mit denselben Bezugszeichen versehen. Gut erkennbar ist der durchgängige Verlauf der beiden Hauptgurte 22, 30 von ihrem jeweiligen freien Ende 26, 32 bis zu einer der Betthalterungen 18. Über diesen Bereich erstreckt sich insbesondere jeweils ein durchgängiger erster Materialstreifen 44, hier in Form eines schmaleren Gurtmaterials. Diese ersten Materialstreifen 44 befinden sich an der Außenseite der Hauptgurte 22, 30 und sind mit weiteren Materiallagen der Hauptgurte 22, 30, die eine größere Breite aufweisen, vernäht. Sie verlaufen von den ersten Enden 24, 32 der Hauptgurte 22, 30 ausgehend durchgängig weiter, jeweils bis zu einem freien Ende einer der Betthalterungen 18.

Außerdem zeigt Fig. 2 einen Schrittgurt 46, der an dem ersten Verbindungsgurt 40 befestigt ist und ein freies Ende 48 sowie ein Polster 50 aufweist. Das freie Ende 48 weist mehrere Ösen auf, so dass der Schrittgurt 48 mit dem Magnetschloss 38 mit den freien Enden 26, 34 der Hauptgurte 22, 30 verbunden werden kann.

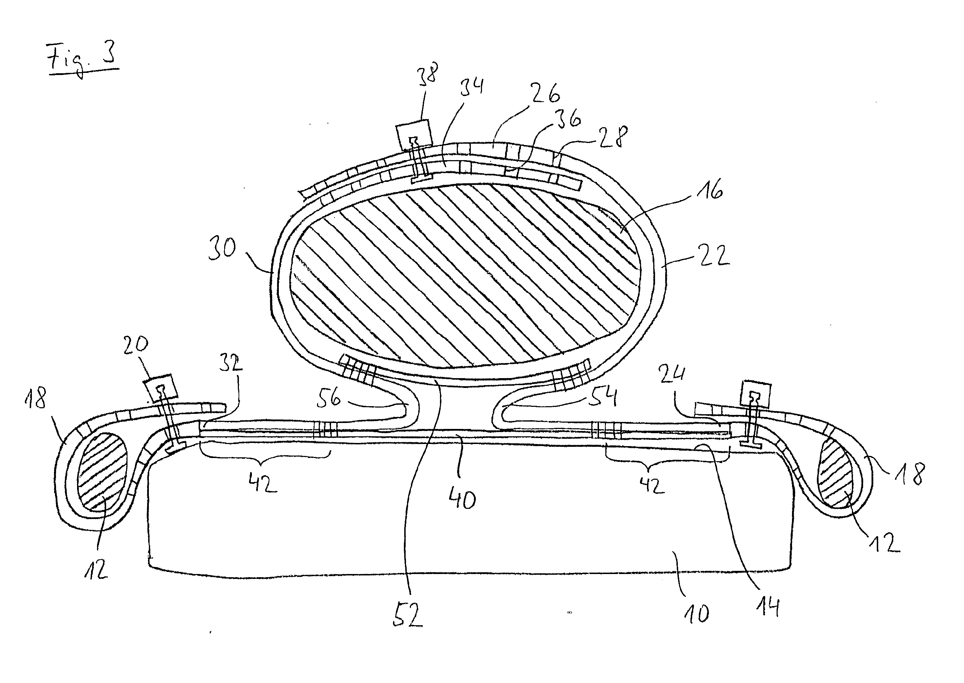

Fig. 3 zeigt ein weiteres Ausführungsbeispiel einer Fixierbandage. Die dem ersten Ausführungsbeispiel entsprechenden Elemente sind mit den gleichen Bezugszeichen versehen wie in den Figuren 1 und 2 und werden nicht erneut erläutert. Auch in dieser Figur sind die Verbindungen zwischen den Betthalterungen 18 und den damit verbundenen Hauptgurten 22, 30 bzw. dem ersten Verbindungsgurt 40 nicht im Einzelnen dargestellt. Sie können auf unterschiedliche Weise hergestellt werden, etwa durch Vernähen überlappender Gurtabschnitte oder durch Verwendung durchgängiger Materialstreifen. Ein Unterschied zum ersten Ausführungsbeispiel ist, dass ein zweiter Verbindungsgurt 52 vorhanden ist, der die beiden Hauptgurte 22, 30 im Rücken des Patienten 16 miteinander verbindet. Er ist im Bereich seiner beiden Enden mit jeweils einem der Hauptgurte 22, 30 vernäht, und zwar in einem in Richtung der Hauptgurte 22, 30 betrachtet größeren Abstand von den ersten Enden 24, 32 der Hauptgurte 22, 30 als die Enden des ersten Verbindungsgurts 40. Somit weisen die Hauptgurte 22, 30 jeweils einen Verbindungsabschnitt 54, 56 auf, der eine Verbindung zwischen den den Patienten umschließenden und den auf der Liegefläche aufliegenden Elementen der Fixierbandage herstellt. Der Verbindungsabschnitt 54 befindet sich links des Patienten 16 und gehört zum linken Hauptgurt 22. Der Verbindungsabschnitt 56 befindet sich rechts des Patienten 16 und gehört zum rechten Hauptgurt 30.

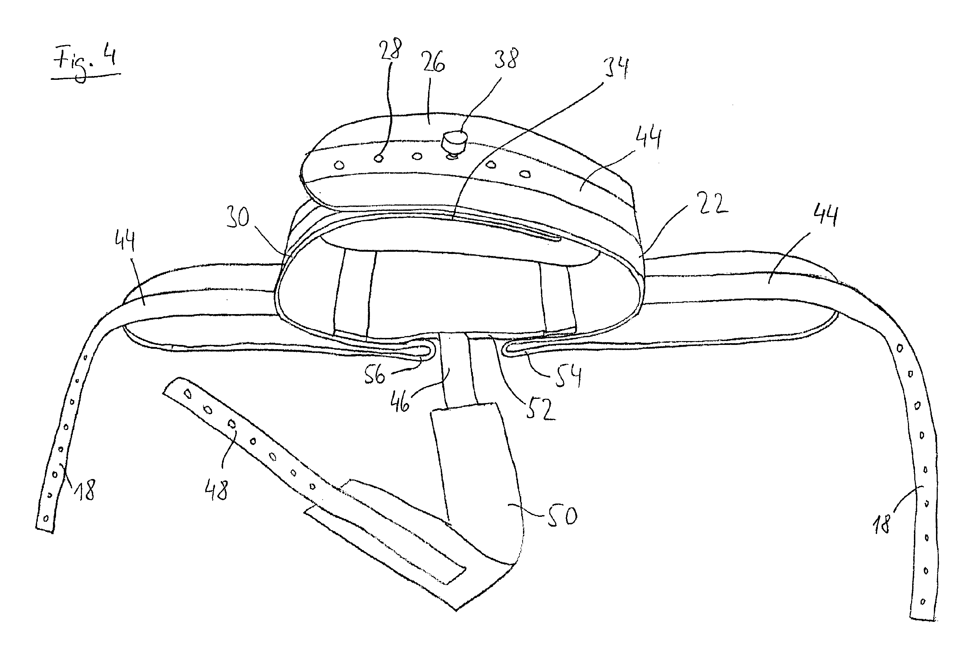

Auch in der Fig. 4 sind die rechten und linken Verbindungsabschnitte 54, 56 erkennbar. Ein Schrittgurt 50 ist in diesem Ausführungsbeispiel an dem zweiten Verbindungsgurt 52 befestigt. Auch in diesem Beispiel hat jeder Hauptgurt 22, 30 einen durchgängigen ersten Materialstreifen 44, der einteilig in die Betthalterungen 18 übergeht. Der ebenfalls vorhandene erste Verbindungsgurt 40 ist in der Figur 4 verdeckt.

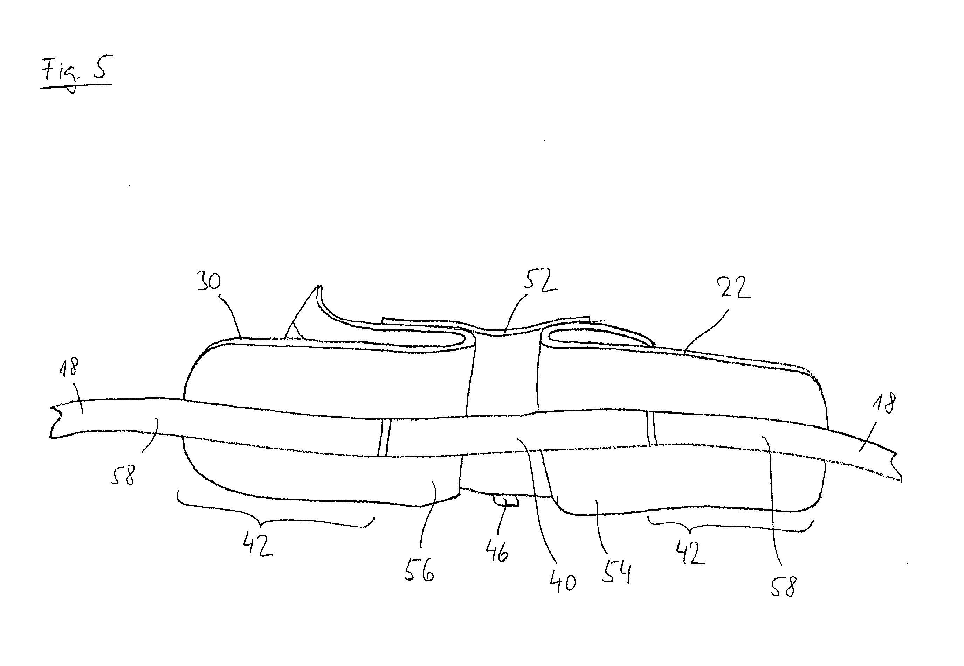

Er ist aus dem Blickwinkel der Figur 5, von der Liegefläche 14 aus gesehen, gut erkennbar und besteht aus einem zweiten Materialstreifen 58, der sich durchgängig von einem freien Ende einer der Betthalterungen 18 bis zu einem freien Ende der gegenüberliegenden Betthalterungen 18 erstreckt.

### Liste der verwendeten Bezugszeichen:

10 Matratze
12 Bettgestell
14 Liegefläche
16 Patient
18 Betthalterung
20 Magnetschloss
22 linker Hauptgurt
24 erstes Ende des linken Hauptgurts
26 freies Ende des linken Hauptgurts
28 Ösen im freien Ende des linken Hauptgurts
30 rechter Hauptgurt
32 erstes Ende des rechten Hauptgurts
34 freies Ende des rechten Hauptgurts
36 Ösen im freien Ende des rechten Hauptgurts
38 Magnetschloss
40 erster Verbindungsgurt
42 erster Gurtabschnitt
44 erster Materialstreifen
46 Schrittgurt
48 freies Ende des Schrittgurts
50 Polster
52 zweiter Verbindungsgurt
54 Verbindungsabschnitt des linken Hauptgurts
56 Verbindungsabschnitt des rechten Hauptgurts
58 zweiter Materialstreifen

## Patentansprüche

1. Fixierbandage zur Fixierung eines Patienten (16) auf einem Bett oder einem sonstigen Lager mit
● zwei Betthalterungen (18) zur Befestigung an einem Bettgestell (12),
● zwei Hauptgurten (22, 30), die jeweils ein erstes Ende (24, 32), das mit einer der Betthalterungen (18) verbunden ist, und ein freies Ende (26, 34) aufweisen, wobei die freien Enden (26, 34) Befestigungseinrichtungen zur lösbaren Verbindung der freien Enden (26, 34) miteinander aufweisen und die Hauptgurte (22, 30) jeweils mindestens einen ersten Materialstreifen (44) aufweisen, der durchgängig vom ersten Ende (24, 32) bis zum freien Ende (26, 34) des Hauptgurts (22, 30) verläuft, und
● einem ersten Verbindungsgurt (40), der die beiden Hauptgurte (22, 30) miteinander verbindet, so dass erste Gurtabschnitte (42) der Hauptgurte (22, 30), die sich jeweils von einem der ersten Enden (24, 32) des jeweiligen Hauptgurts (22, 30) bis zu dem ersten Verbindungsgurt (40) erstrecken, und der erste Verbindungsgurt (40) mit Hilfe der Betthalterungen (18) über eine Liegefläche (14) des Betts gespannt und die freien Enden (26, 34) der beiden Hauptgurte (22, 30) um den Leib des Patienten (16) herumgeführt und miteinander verbunden werden können.

2. Fixierbandage nach Anspruch 1, **dadurch gekennzeichnet, dass** die Hauptgurte (22, 30) eine erste Breite aufweisen und der mindestens eine erste Materialstreifen ein erstes Gurtmaterial mit der ersten Breite ist.

3. Fixierbandage nach Anspruch 1 oder 2, **dadurch gekennzeichnet, dass** der erste Verbindungsgurt (40) die gleiche Breite aufweist wie die Hauptgurte (22, 30).

4. Fixierbandage nach Anspruch 2 oder 3, **dadurch gekennzeichnet, dass** der erste Verbindungsgurt (40) einen Streifen des ersten Gurtmaterials aufweist.

5. Fixierbandage nach einem der Ansprüche 1 bis 4, **dadurch gekennzeichnet, dass** der mindestens eine erste Materialstreifen (44) durchgängig von einem freien Ende (26, 34) eines der Hauptgurte (22, 30) bis zu einem freien Ende einer der Betthalterungen (18) verläuft.

6. Fixierbandage nach einem der Ansprüche 1 bis 5, **dadurch gekennzeichnet, dass** der erste Verbindungsgurt (40) mindestens einen zweiten Materialstreifen (58) aufweist, der durchgängig bis zu einer der beiden Betthalterungen (18) verläuft.

7. Fixierbandage nach Anspruch 6, **dadurch gekennzeichnet, dass** der zweite Materialstreifen (58) von einem Befestigungspunkt des ersten Verbindungsgurts (40) an einem Hauptgurt (22, 30) entlang dieses Hauptgurts (22, 30) verläuft und mit diesem vernäht ist.

8. Fixierbandage nach einem der Ansprüche 1 bis 7, **dadurch gekennzeichnet, dass** ein zweiter Verbindungsgurt (52) vorhanden ist, der die beiden Hauptgurte (22, 30) im Rücken des Patienten (16) miteinander verbindet.

9. Fixierbandage nach Anspruch 8, **dadurch gekennzeichnet, dass** die Enden des zweiten Verbindungsgurts (52) jeweils in einem entlang der Hauptgurte (22, 30) betrachtet größeren Abstand von den ersten Enden (24, 32) des jeweiligen Hauptgurts (22, 30) entfernt an dem Hauptgurt (22, 30) befestigt sind als die Enden des ersten Verbindungsgurts (40).

10. Fixierbandage nach Anspruch 8 oder 9, **dadurch gekennzeichnet, dass** der zweite Verbindungsgurt (52) die gleiche Breite aufweist wie die beiden Hauptgurte (22, 30).

11. Fixierbandage nach einem der Ansprüche 8 bis 10, **dadurch gekennzeichnet, dass** der zweite Verbindungsgurt (52) mindestens ein Stück des ersten Gurtmaterials aufweist.

12. Fixierbandage nach einem der Ansprüche 8 bis 11, **dadurch gekennzeichnet, dass** der erste Verbindungsgurt (40) eine zweite Breite aufweist, die geringer ist als die erste Breite.

13. Fixierbandage nach Anspruch 12, **dadurch gekennzeichnet, dass** der zweite Materialstreifen (58) ein zweites Gurtmaterial mit der zweiten Breite ist.

14. Fixierbandage nach Anspruch 13, **dadurch gekennzeichnet, dass** die beiden Betthalterungen (18) die zweite Breite aufweisen und mindestens ein Stück des zweiten Gurtmaterials.

15. Fixierbandage nach einem der Ansprüche 6 bis 14, **dadurch gekennzeichnet, dass** der zweite Materialstreifen (58) durchgängig bis zu einem freien Ende einer der Betthalterungen (18) verläuft.

16. Fixierbandage nach einem der Ansprüche 1 bis 15, **dadurch gekennzeichnet, dass** an dem ersten Verbindungsgurt (40) oder an dem zweiten Verbindungsgurt (52) ein Schrittgurt (46) befestigt ist oder Oberschenkelmanschetten befestigt sind.

17. Fixierbandage nach einem der Ansprüche 1 bis 16, **dadurch gekennzeichnet, dass** an den Hauptgurten (22, 30) Befestigungseinrichtungen zur Anbringung eines Brust- oder Schultergurts ausgebildet sind.

18. Fixierbandage nach einem der Ansprüche 1 bis 17, **dadurch gekennzeichnet, dass** ein Verbindungsbereich zwischen einem der Hauptgurte (22, 30) und einem der Verbindungsgurte (40, 52) mit einem Materialstück verstärkt ist, das mit dem jeweiligen Hauptgurt (22, 30) und dem jeweiligen Verbindungsgurt (40, 52) vernäht ist.
